# EUROPÄISCHE PATENTSCHRIFT

(11) **EP 0 691 337 B1**
(45) Veröffentlichungstag und Bekanntmachung des Hinweises auf die Patenterteilung: **10.11.1999**
(21) Anmeldenummer: 95110138.5
(22) Anmeldetag: 29.06.1995
(51) Int. Cl.: C07D 309/18

(54) **Verfahren zur Herstellung von 3,4-Dihydro-2H-pyran**
Process for the preparation of 3,4-dihydro-2H-pyran
Procédé de préparation de 3,4-dihydro-2H-pyranne

(30) Priorität: 07.07.1994 DE 4423933
(43) Veröffentlichungstag der Anmeldung: 10.01.1996
(73) Patentinhaber: BASF AKTIENGESELLSCHAFT, 67056 Ludwigshafen (DE)
(72) Erfinder: Schröder, Jürgen, Dr., D-67071 Ludwigshafen (DE); Ebel, Klaus, Dr., D-68623 Lampertheim (DE)

(56) Entgegenhaltungen:
- FR-A- 887 493
- GB-A- 858 626
- GB-A- 1 017 313

## Beschreibung

Die vorliegende Erfindung betrifft ein Verfahren zur Herstellung von 3,4-Dihydro-2H-pyran durch dehydratisierende Umlagerung von Tetrahydrofurfurylalkohol in der Wirbelschicht an oxidischen Katalysatoren.

Aus J. Am. Chem. Soc., 68 (1946), Seite 1646 bis 1648 ist ein Verfahren zur Herstellung von 3,4-Dihydro-2H-pyran durch Umsetzung von Tetrahydrofurfurylalkohol bei 375°C an aktiviertem Aluminiumoxid bekannt. Die Nichtbeachtung der komplizierten Anfahrvorschrift führt zu Temperaturspitzen von bis zu 525°C und zur Desaktivierung des Katalysators.

Aus Organic Syntheses, Collective Vol. 3, Seite 276 bis 277 ist ein Verfahren zur Herstellung von 3,4-Dihydro-2H-pyran durch Umsetzung von Tetrahydrofurfurylalkohol in einem Festbettreaktor bekannt. Die Standzeiten des eingesetzten aktivierten Aluminiumoxids werden mit 12 bis 16 Stunden angegeben. Auf dem Katalysator entstehen braune teerartige Ablagerungen.

In GB-A-10 17 313 werden zur Herstellung von 3,4-Dihydro-2H-pyran aus Tetrahydrofurfurylalkohol Vanadiumoxid bzw. Molybdänoxid auf aktiviertem Aluminiumoxid als Katalysatoren vorgeschlagen. Die Ausbeuten betragen bis zu 76 %.

Der vorliegenden Erfindung lag daher die Aufgabe zugrunde, den zuvor genannten Nachteilen abzuhelfen.

Demgemäß wurde ein neues und verbessertes Verfahren zur Herstellung von 3,4-Dihydro-2H-pyran durch Umsetzung von Tetrahydrofurfurylalkohol an festen oxidischen Katalysatoren gefunden, welches dadurch gekennzeichnet ist, daß man die Umsetzung in der Gasphase im Wirbelbett bei Temperaturen von 150 bis 350°C und Drücken von 0,001 bis 50 bar durchführt.

Das erfindungsgemäße Verfahren läßt sich wie folgt durchführen:

Tetrahydrofurfurylalkohol kann gasförmig mit einem inerten Trägergas, bevorzugt Stickstoff, über den auf Reaktionstemperatur von 150 bis 350°C, bevorzugt 200 bis 300°C, erhitzten Wirbelbettkatalysator geleitet werden. Der Reaktionsdruck kann in weiten Grenzen variiert werden und beträgt in der Regel 0,001 bis 50 bar, bevorzugt 0,01 bis 10 bar, besonders bevorzugt Normaldruck (Atmosphärendruck). Das den Wirbelbettreaktor verlassende Gasgemisch kann kondensiert, die organische Phase abgetrennt und fraktioniert werden.

Als feste oxidische Katalysatoren eignen sich Oxide der Gruppen IIa, IIIa, IIb, IIIb, IVb, Vb, VIb und VIIb des Periodensystems der Elemente, des Eisens, Kobalts, Nickels, Cers, Praseodyms oder deren Gemische, bevorzugt Oxide von Magnesium, Calcium, Aluminium, Zink, Titan, Zirkon, Vanadin, Chrom, Molybdän, Mangan, Eisen, Kobalt, Nickel, Cer oder deren Gemische, besonders bevorzugt Oxide von Magnesium, Calcium, Aluminium, Zink, Titan, Zirkon, Mangan, Eisen, Kobalt, Nickel oder deren Gemische insbesondere Aluminiumoxid wie gamma-Aluminiumoxid. Diese festen oxidischen Katalysatoren können gegebenenfalls mit Phosphorsäure getränkt sein.

Im Vergleich zu den bekannten Verfahren liefert das erfindungsgemäße Verfahren auf einfacherem und wirtschaftlicherem Weg 3,4-Dihydro-2H-pyran. Die langen Standzeiten der eingesetzten Katalysatoren sind überraschend.

3,4-Dihydro-2H-pyran ist eine wertvolle Schutzgruppe für Alkohole.

### Beispiele

### Beispiel 1

Stündlich wurden 51 g Tetrahydrofurfurylalkohol verdampft und mit 110 Nl Stickstoff durch einen auf 250°C erhitzten Wirbelbettreaktor geleitet. Der Wirbelbettreaktor war mit 500 ml eines Katalysators aus gamma-Aluminiumoxid gefüllt. Die Reaktionsgase wurden anschließend kondensiert und die Phasen getrennt. Man erhielt stündlich 32,3 g (77 %) 3,4-Dihydro-2H-pyran. Der Katalysator war nach 54 Stunden unverändert aktiv. Verfärbungen, die auf eine beginnende Desaktivierung des Katalysators hindeuten würden, traten nicht auf.

### Beispiel 2

Man verfuhr wie in Beispiel 1, setzte jedoch 500 ml eines Katalysators aus 95 % gamma-Aluminiumoxid und 5 % Phosphorsäure ein. Man erhielt stündlich 30,2 g (72 %) 3,4-Dihydro-2H-pyran. Der Katalysator war nach 32 Stunden unverändert aktiv. Verfärbungen, die auf eine beginnende Desaktivierung des Katalysators hindeuten würden, traten nicht auf.

## Patentansprüche

1. Verfahren zur Herstellung von 3,4-Dihydro-2H-pyran durch Umsetzung von Tetrahydrofurfurylalkohol an festen oxidischen Katalysatoren, dadurch gekennzeichnet, daß man die Umsetzung in der Gasphase im Wirbelbett bei Temperaturen von 150 bis 350°C und Drücken von 0,001 bis 50 bar durchführt.

2. Verfahren zur Herstellung von 3,4-Dihydro-2H-pyran nach Anspruch 1, dadurch gekennzeichnet, daß man als feste oxidische Katalysatoren Oxide der Gruppen IIa, IIIa, IIb, IIIb, IVb, Vb, VIb und VIIb des Periodensystems der Elemente, des Eisens, Kobalts, Nickels, Cers, Praseodyms oder deren Gemische einsetzt.

3. Verfahren zur Herstellung von 3,4-Dihydro-2H-pyran nach Anspruch 1, dadurch gekennzeichnet, daß man als feste oxidische Katalysatoren Oxide von Magnesium, Calcium, Aluminium, Zink, Titan, Zirkon, Vanadin, Chrom, Molybdän, Mangan, Eisen, Kobalt, Nickel, Cer oder deren Gemische einsetzt.

4. Verfahren zur Herstellung von 3,4-Dihydro-2H-pyran nach Anspruch 1, dadurch gekennzeichnet, daß man als feste oxidische Katalysatoren Oxide von Magnesium, Calcium, Aluminium, Zink, Titan, Zirkon, Mangan, Eisen, Kobalt, Nickel oder deren Gemische einsetzt.

5. Verfahren zur Herstellung von 3,4-Dihydro-2H-pyran nach Anspruch 1, dadurch gekennzeichnet, daß man als feste oxidische Katalysatoren Aluminiumoxid einsetzt.

6. Verfahren zur Herstellung von 3,4-Dihydro-2H-pyran nach Anspruch 1, dadurch gekennzeichnet, daß man die Umsetzung bei Temperaturen von 200 bis 300°C durchführt.

7. Verfahren zur Herstellung von 3,4-Dihydro-2H-pyran nach Anspruch 1, dadurch gekennzeichnet, daß man die Umsetzung bei Drücken von 0,01 bis 10 bar durchführt.

## Claims

1. A process for preparing 3,4-dihydro-2H-pyran by reaction of tetrahydrofurfuryl alcohol over solid oxidic catalysts, wherein the reaction is carried out in the gas phase in a fluidized bed at from 150 to 350°C and pressures of from 0.001 to 50 bar.

2. A process for preparing 3,4-dihydro-2H-pyran as claimed in claim 1, wherein the solid oxidic catalysts used are oxides of groups IIa, IIIa, IIb, IIIb, IVb, Vb, VIb and VIIb of the Periodic Table of the Elements, of iron, cobalt, nickel, cerium, praseodymium or mixtures thereof.

3. A process for preparing 3,4-dihydro-2H-pyran as claimed in claim 1, wherein the solid oxidic catalysts used are oxides of magnesium, calcium, aluminum, zinc, titanium, zirconium, vanadium, chromium, molybdenum, manganese, iron, cobalt, nickel, cerium or mixtures thereof.

4. A process for preparing 3,4-dihydro-2H-pyran as claimed in claim 1, wherein the solid oxidic catalysts used are oxides of magnesium, calcium, aluminum, zinc, titanium, zirconium, manganese, iron, cobalt, nickel or mixtures thereof.

5. A process for preparing 3,4-dihydro-2H-pyran as claimed in claim 1, wherein the solid oxidic catalyst used is aluminum oxide.

6. A process for preparing 3,4-dihydro-1H-pyran as claimed in claim 1, wherein the reaction is carried out at from 200 to 300°C.

7. A process for preparing 3,4-dihydro-2H-pyran as claimed in claim 1, wherein the reaction is carried out at pressures of from 0.01 to 10 bar.

## Revendications

1. Procédé de préparation de 3,4-dihydro-2H-pyrane par réaction d'alcool tétrahydrofurfurylique sur des catalyseurs oxydes solides, caractérisé en ce que l'on mène la réaction en phase gazeuse en lit fluidisé, à des températures de 150-350°C et sous des pressions de 0,001-50 bar.

2. Procédé de préparation de 3,4-dihydro-2H-pyrane selon la revendication 1, caractérisé en ce que l'on utilise en tant que catalyseurs oxydes solides, des oxydes des groupes IIA, IIIA, IIB, IIIB, IVB, VB, VIB et VIIB de la classification périodique des éléments, de fer, de cobalt, de nickel, de cérium, de praséodyme ou leurs mélanges.

3. Procédé de préparation de 3,4-dihydro-2H-pyrane selon la revendication 1, caractérisé en ce que l'on utilise en tant que catalyseurs oxydes solides, des oxydes de magnésium, de calcium, d'aluminium, de zinc, de titane, de zirconium, de vanadium, de chrome, de molybdène, de manganèse, de fer, de cobalt, de nickel, de cérium, de praséodyme ou leurs mélanges.

4. Procédé de préparation de 3,4-dihydro-2H-pyrane selon la revendication 1, caractérisé en ce que l'on utilise en tant que catalyseurs oxydes solides, des oxydes de magnésium, de calcium, d'aluminium, de zinc, de titane, de zirconium, de manganèse, de fer, de cobalt, de nickel, ou de mélanges.

5. Procédé de préparation de 3,4-dihydro-2H-pyrane selon la revendication 1, caractérisé en ce que l'on utilise de l'oxyde d'aluminium en tant que catalyseurs oxydes solides.

6. Procédé de préparation de 3,4-dihydro-2H-pyrane selon la revendication 1, caractérisé en ce que l'on mène la réaction à des températures de 200-300°C.

7. Procédé de préparation de 3,4-dihydro-2H-pyrane selon la revendication 1, caractérisé en ce que l'on mène la réaction sous des pressions de 0,01-10 bar.
